# EUROPÄISCHE PATENTANMELDUNG

(11) **EP 2 347 797 A2**
(43) Veröffentlichungstag der Anmeldung: **27.07.2011**
(21) Anmeldenummer: 10197053.1
(22) Anmeldetag: 27.12.2010
(51) Int. Cl.: A61Q 19/00, A61Q 19/08, A61Q 19/10, A61K 8/19

(54) **Kosmetisches Verfahren**

(30) Priorität: 22.01.2010 DE 102010001156
(71) Anmelder: Simply Water GmbH, 93055 Regensburg (DE)
(72) Erfinder: Philipps, André, 93059 Regensburg (DE)
(74) Vertreter: Bublak, Wolfgang

(57) **Zusammenfassung**

Die vorliegende Erfindung betrifft ein kosmetisches Verfahren zur Erzielung eines Hautpflegevorteils, ausgewählt aus: Behandeln/Verhindern von faltiger, herunterhängender und/oder gealterter Haut; Verstärken der Collagenabscheidungen in der Haut; Verbesserung der Hauttextur und/oder Glätte und/oder Festigkeit; Reinigung der Haut, wobei das Verfahren das Auftragen einer lokal anzuwendenden Zusammensetzung, umfassend physikalisch behandeltes Wasser, auf die Haut umfasst, wobei das physikalisch behandelte Wasser mittels eines Influenzverfahrens gewonnen wird.

Darüber hinaus betrifft die vorliegende Erfindung die Verwendung von physikalisch behandeltem Wasser zur Herstellung einer lokal anzuwendenden Zusammensetzung zur Verwendung in einem Verfahren nach einem der vorhergehenden Ansprüche sowie ein Verfahren zur Übertragung eines Elektronenmangels auf die Haut zur physikalischen Behandlung der Haut zur Erzielung eines Hautpflegevorteils.

Die Erfindung bietet eine erhebliche Verbesserung bei der Hautpflege und Unterstützung der natürlichen Hautfunktionen.

## Beschreibung

Die vorliegende Erfindung betrifft ein kosmetisches Verfahren zur Erzielung eines Hautpflegevorteils, die Verwendung von physikalisch behandeltem Wasser zur Verwendung in einem solchen Verfahren sowie ein Verfahren zur Übertragung eines Elektronenmangels auf die Haut.

Die Haut des Säugetieres, insbesondere Menschen, unterliegt fortgesetzten Verschlechterungen durch dermatologische Störung, z. B. durch Umwelteinflüsse (Wind, Klimaanlage, Zentralheizung) oder durch den normalen Alterungsprozess (Chronoaltern) welcher durch das Aussetzen der Haut der Sonne beschleunigt wird (Photoaltern). In den letzten Jahren haben die Anforderungen an kosmetische Zusammensetzungen und kosmetische Verfahren zum Verbessern des Aussehens und Zustandes der Haut stark zugenommen. Gleichzeitig aber wünschen sich die Konsumenten verstärkt Verfahren zur Erzielung eines Hautvorteils, der nicht von Nebenwirkungen begleitet wird.

Die Verbraucher suchen zunehmend nach kosmetischen Verfahren, die die sichtbaren Anzeichen von zeitlich und durch Licht gealterter Haut, wie Falten, Linien, Herunterhängen, Hyperpigmentierung und Altersflecken behandeln und verzögern. Gleichzeitig wird von einem kosmetischen Verfahren erwartet, dass es die Haut reinigt. Ein erheblicher Teil der Hautalterungsvorgänge wird u.a. durch die Einlagerungen von Umweltfaktoren wie Staub oder Allergenen hervorgerufen, sodass eine gründliche Entfernung dieser extern zugefügten Stoffe die Alterung der Haut verlangsamen kann. Darüber hinaus lagern sich, insbesondere im zunehmenden Lebensalter, in der Haut Stoffwechselendprodukte bzw. andere schädliche Substanzen ab, sodass im Rahmen einer umfassenden kosmetischen Hautbehandlung eine Entfernung dieser Stoffe ebenfalls angestrebt wird.

Die Verbraucher suchen auch häufig nach kosmetischen Verfahren, die über die reine Verminderung von Alterserscheinungen hinweg die Haut zusätzlich verbessern. So hat das Konzept der "empfindlichen Haut" die Nachfrage der Verbraucher für kosmetische Produkte bzw. Verfahren, die das Aussehen und den Zustand von empfindlicher, trockener und/oder schuppiger Haut verbessern und/oder gereizte Haut mindern, ansteigen lassen. Ebenso wünschen sich die Verbraucher kosmetische Verfahren, die Flecken, Pickel oder Schönheitsfehler behandeln.

Kosmetische Verfahren, die diese Erwartungen des Verbrauchers erfüllen, sind derzeit nicht zugänglich.

Ausgehend von diesem Stand der Technik ist es die Aufgabe der vorliegenden Erfindung, ein kosmetisches Verfahren bzw. die Verwendung von physikalisch behandeltem Wasser zur Verwendung in einem kosmetischen Verfahren bereit zustellen, die die geschilderten Nachteile im Stand der Technik überwindet.

Gelöst wird diese Aufgabe mit den Merkmalen des Patenanspruchs 1. Vorteilhafte Aus- und Weiterbildungen, welche einzeln oder in Kombination miteinander eingesetzt werden können, sind Gegenstand der Unteransprüche.

Die vorliegende Erfindung löst die gestellte Aufgabe dadurch, dass sie ein kosmetisches Verfahren zur Erzielung eines Hautpflegevorteils, ausgewählt aus: Behandeln/Verhindern von faltiger, herunterhängender und/oder gealterter Haut; Verstärken der Collagenabscheidungen in der Haut; Verbesserung der Hauttextur und/oder Glätte und/oder Festigkeit; Reinigung der Haut zur Verfügung stellt, wobei das Verfahren das Auftragen einer lokal anzuwendenden Zusammensetzung, umfassend physikalisch behandeltes Wasser, auf die Haut umfasst, wobei das physikalisch behandelte Wasser mittels eines Influenzverfahrens gewonnen wird.

Dadurch wird gegenüber dem Stand der Technik eine erhebliche Verbesserung erzielt. Das erfindungsgemäße Verfahren bekämpft wirksam altersbedingte, genetisch oder umweltbedingte Veränderungen der Haut, ohne aber die Funktion der Haut zu stören.

Das erfindungsgemäße Verfahren sowie die Verwendung von physikalisch behandeltem Wasser zur Herstellung einer lokal anzuwendenden Zusammensetzung zur Verwendung in einen solchen Verfahren stellt somit Antialterungsvorteile bereit, die sich in der Förderung von glatter und geschmeidiger Haut mit verbesserter Elastizität und einem verminderten oder verzögerten Aussehen von Falten und gealterter Haut mit verbesserter Hautfarbe ergeben. Eine allgemeine Verbesserung im Aussehen, in der Textur und dem Zustand, insbesondere bezüglich Strahlen, Klarheit und allgemein jugendliches Aussehen von Haut, wird erreicht. Die erfindungsgemäßen Verfahren/Verwendungen sind auch vorteilhaft zum Glätten und Beruhigen von empfindlicher und/oder gereizter Haut. Somit stellt das erfindungsgemäße Verfahren vorteilhafterweise einen breiten Bereich von Hautpflegevorteilen bereit.

Der hierhin verwendeter Begriff "Behandeln" schließt in seinem Umfang Vermindern, Verzögern und/oder Verhindern der vorstehend erwähnten Hautzustände, wie faltige, gealterte, lichtgeschädigte und/oder gereizter Haut und allgemeines Verstärken der Qualität der Haut und Verbessern ihres Aussehens die ihre Textur durch Verhindern oder Vermindern von Faltenbildung und Erhöhung von Biegsamkeit, Festigkeit, Glätte, Geschmeidigkeit und Elastizität der Haut ein. Die kosmetischen Verfahren und Anwendungen von physikalisch behandeltem Wasser gemäß der Erfindung können zum Behandeln von Haut, die bereits in einem faltigen, gealterten, lichtgeschädigten und gereizten Zustand ist, oder zum Behandeln von jugendlicher Haut, und die vorstehend erwähnten verschlechternden Veränderungen aufgrund des normalen Alterungs-/Lichtalterungsprozesses zu verhindern oder zu vermindern, verwendbar sein.

Das erfindungsgemäße kosmetische Verfahren wird wie folgt durchgeführt. Zum besseren Verständnis werden die physikalischen Grundlagen untenstehend kurz erläutert.

In dem kosmetischen Verfahren erfolgt die Behandlung der Haut mittels einer elektrostatischen Ladung - das elektrochemisch behandelte Wasser dient hierbei zur Übertragung der Ladung, vergleichbar einer transportablen Batterie.

Der Begriff "kosmetisches Verfahren" ist in einem weiten Sinne zu verstehen, und umfasst die Körper- und Schönheitspflege, bzw. die Erhaltung, Wiederherstellung oder gar Verbesserung der Schönheit des menschlichen Körpers, insbesondre der Haut einschließlich der Schleimhäute wie z.B. des Mund-Rachenraumes als auch der Augenoberfläche. Beispielsweise kann das kosmetische Verfahren auch zur Pflege der Mundhöhle in der Verwendung als Mundwasser oder zur Pflege des Auges in der Verwendung als Augenwasser eingesetzt werden.

Bei dem Verfahren beruht die Wirkung des physikalisch behandelten Wassers auf der Anregung des Wassermoleküls selbst. Die Wassermoleküle befinden sich in einem Clusterverband, der sich aufgrund der elektrostatischen Dipolstruktur des Wassermoleküls ausbildet.

Mit der modernen NMR-Analyse (Nuclear Magnetic Resonance = Spinkemresonanztomography) kann gezeigt werden, dass Trinkwasser eine Clustergröße von 10 bis 13 H₂O-Molekülen hat. Gesundes Quell- und Bachwasser, Schmelzwasser und Regenwasser in Gebieten ohne Luftverschmutzung hat kleinere Cluster mit 6 bis 8 Molekülen. Es ist in der Wissenschaft unumstritten, dass Wasser eine umso höhere Lösungskraft und Vitalität hat, je kleiner die Cluster sind. Ein Grund dafür kann sein, dass bei kleineren Clustern die nach außen reaktive Oberfläche der Wassercluster wesentlich größer ist als bei Wasser mit größeren Cluster.

Es konnte nun überraschenderweise gezeigt werden, dass ein mittels Influenzverfahren behandeltes Wasser eine deutlich kleinere Clustergröße aufweist als konventionelles Leitungswasser. Es wird davon ausgegangen, dass Wasser mit kleinerer Clustergröße besser vom Körper aufgenommen werden kann. Weiterhin werden durch die Durchführung einer Influenz Wassermoleküle elektrisch entladen und die erzeugten Ladungsträger im Clusterverband durch ständigen Austausch stabilisiert (Grotthusmechanismus). Das derart elektrisch entladene Wasser kann desinfizierend wirken, da es im Stande ist, Zellstrukturen zu denaturieren bzw. die Elektronentransportmechanismen von Mikroorganismen irreversibel zu stören. Darüber hinaus ist es in der Lage, in oxidative Prozesse der Haut einzugreifen, die für die Hautalterung verantwortlich sind.

Wassermoleküle sind bekanntermaßen Dipole, deren entgegengesetzt geladene Enden sich anziehen. So entstehen zunächst Dimere, die seit 1961 als Zundel-Kation bezeichnet werden. Diese lagern sich zu größeren Verbänden zusammen, den sog. Clustern. Cluster sind eine Untergruppe der van-der-Waals-Körper, da diese durch London-van-der-Waals-Kräfte zusammengehalten werden. Dabei ist die Größe der Cluster u.a. abhängig vom Ort im Wasser, an dem sie sich befinden.

Wesentlich für van-der-Waals-Cluster ist deren Eigenschaft, dass Elektrone nicht mehr an die Orbitale und Schalen ihrer Mutter-Atome/Moleküle gebunden sind. Sie sind nach der Schrödinger-Gleichung (1926) statistisch im Cluster verteilt und können frei im Cluster-Verbund vagabundieren. In festen van-der-Waals-Clustern (wie z.B. Metallen) bezeichnet man sie in ihrer Gesamtheit als Elektronengas, z.B. verantwortlich für die elektrische Leitfähigkeit von Metallen.

Dem Wasser wird durch den oben beschriebenen Prozess Elektronen entzogen. Ein Elektronenmangel in den Clustern (unsauber als Proton bezeichnet) führt nicht zur Instabilität des Clusterverbandes, sondern wird über den GrotthusMechanismus (1820) durch sog. Protonenhopping ausgeglichen.

Das erfindungsgemäße Verfahren beruht auf elektrostatischer Induktion oder Influenz. Dabei führt eine Bewegung im elektrischen Feld zur Trennung von Ladungen, der Influenz.

Die Gewinnung des behandelten Wassers mittels Influenzverfahren wird wie folgt durchgeführt:
Cluster-Wasser, dessen Leitfähigkeit durch den Zusatz von Kochsalz auf einen Sollwert eingestellt ist, fließt in einem elektrostatischen Feld (ruhendes elektrisches Feld), das durch eine Anode und eine Kathode gebildet wird.

Im ersten Schritt kommt es zur Ausrichtung der Ladungen und freien Elektronen im elektrischen Feld.

Durch die Bewegung und die daraus folgende Influenz werden in einem zweiten Schritt die Ladungen getrennt und abgeführt

Die Fraktion mit einem Mangel an Elektronen wird als zu nutzendes Konzentrat abgeführt und gesammelt, das Ergebnis ist eine de-elektronisierte (positiv geladene) Fraktion. Bei Bedarf kann zusätzlich die negativ geladene Fraktion mit eingesetzt werden.

Man erhält ein elektrostatisch positiv geladenes Wasser. Es hat einen hohen Bedarf, die ungeladenen Positionen in den Clustern zu füllen. Im Kontakt mit elektronenreichen Oberflächen gibt es einen elektrischen Schlag, der zum Ladungsausgleich führt.

Messung und Nachweis der positiven elektrostatischen Ladung

Problematisch ist, dass es zurzeit kein Messverfahren gibt, eine positive elektrostatische Ladung im Wasser zu messen. Mangels besserer Methoden und aus historischen Gründen behilft man sich daher mit der DPD-Methode, d.h. der Messung der oxidativen Veränderung des Farbstoffes DPD durch Elektronenentzug durch das zu messende Oxidationsmittel. Je nach verwendetem Messgerät wird die "Oxidationskraft" als Konzentration von Wasserstoffperoxid (H₂O₂), Ozon (O₃) oder freiem Chlor ausgedrückt. Die "Chlormessung" ist die am weitesten verbreitete Methode. Sie dient auch zurzeit als einfache vor-Ort-Methode dazu, Konzentrationen zu bestimmen und einzustellen.

Das DPD (N,N-Diethyl-1,4-phenylendiamin) ist ein Farbkomplex, der bei Abgabe von Elektronen von farblos in Rot wechselt, bei der Wiederaufnahme von Elektronen dann wieder von Rot in farblos. Es findet keine chemische Verbindung des Farbstoffes mit Chlor, Ozon oder Wasserstoffperoxid statt, sondern diese entziehen aufgrund ihrer Eigenschaft als Oxidantien lediglich Elektronen.

Das bei dem kosmetischen Verfahren verwendete mittels Influenzverfahren behandelte Wasser hat nach dieser Messmethode eine elektrostatische Ladung, die einem Äquivalent an freiem Chlor von etwa 150 ppm bis 170 ppm entspricht.

Da das Verfahren entfernte Ähnlichkeit mit bekannten Elektrolyseverfahren hat, sollte untersucht werden, ob der Elektronenentzug zu den für Elektrolyse typischen ungepaarten Elektronen, d.h. zur Radikal-Bildung führt. Dazu wurde mittels der Elektronen-Spin-Resonanz-Methode nach Molekülen oder Ionen mit ungepaarten Elektronen im aktivierten Wasser gesucht. Mit der Elektronen-Spin-Resonanz (ESR) lassen sich Moleküle oder Ionen mit Gesamtelektronenspin ungleich Null nachweisen und quantifizieren. Das Verfahren ist auch unter dem Namen EPR (für Electron Paramagnetic Resonance) bekannt.

Im direkten Nachweisverfahren konnten keine ungepaarten Elektronen nachgewiesen werden. Die Menge der ungepaarten Elektronen lag unter der Nachweisgrenze von 1010 Spin/Gauß.

Im Anschluss wurde unter Verwendung zweier sogenannter Spin Traps versucht, ungepaarte Elektronen nachzuweisen. Verwendet wurden DMPO und PBN. Diese Substanzen reagieren auf Moleküle mit ungepaarten Elektronen und ergeben entsprechende Resonanzen.

Auch mit den Spin Traps konnten keine ungepaarten Elektronen nachgewiesen werden.

Durch dergestalt produzierte Elektronenarmut werden die Wassercluster elektrisch entladen. Es entstehen positiv geladene Wassercluster, die als Elektronenakzeptoren fungieren, der sogenannte Elektronenhunger. Dieser sättigt sich an einem Elektronendonator, z. B. jede Form von Einzellern oder einer reduzierenden Substanzen.

Gemäß der Erfindung wird das physikalisch behandelte Wasser mittels eines Influenzverfahrens gewonnen. Ein dergestalt behandeltes Wasser weist physikalische Eigenschaften auf, die für die physikalische Hautbehandlungen von großem Vorteil sind.

Besonders wirkungsvoll ist das Verfahren dann, wenn das physikalisch behandelte Wasser ein Elektronenmangel aufweist. Ein solcher Elektronenmangel sättigt sich an einem Elektronendonator.

Es hat sich als besonders vorteilhaft erwiesen, wenn das physikalisch behandelte Wasser auf die Haut aufgetragen und/oder eingerieben und/oder mittels Eintauchen des Körpers bzw. Körperteils in das physikalisch behandelte Wasser erfolgt. Insbesondere beim Eintauchen des Körpers in das physikalisch behandelte Wasser, z. B. im Rahmen eines Bades, wird eine besonders umfassende Behandlung der Haut erreicht. Je nach Bedarf können der Flüssigkeit beim Bad weitere pflegende Substanzen zugesetzt werden. Auch kann die Haut mechanisch stimuliert werden, z. B. durch eine Massagedusche oder einen Whirlpool oder eine manuelle Massage, um die Wirkung des Verfahrens auf die Haut weiter zu verstärken.

Von Vorteil ist es weiterhin, wenn das Influenzverfahren bei einer Stromdichte von 0,5 bis 10 W/cm² durchführbar ist.

Von Vorteil ist es weiterhin, wenn das zur Herstellung des physikalisch behandelten Wassers das Influenzverfahren folgende Schritte umfasst: Einbringen des zu behandelnden Wassers in ein galvanisches Feld, Ausrichtung der Ladungen und freien Elektronen im elektrischen Feld, Trennung der Ladungen durch Bewegung und der daraus resultierenden Influenz und Sammlung und Abführung der deelektronisierten, positiv geladenen Fraktion.

Ein weiterer Aspekt der Erfindung betrifft die Verwendung von physikalisch behandeltem Wasser zur Herstellung einer lokal anzuwendenden Zusammensetzung zur Verwendung in einem Verfahren zur Erzielung eines Hautpflegevorteils, ausgewählt aus, Behandeln/Verhindern von faltiger, herunterhängender und/oder gealterten Haut; Verstärken der Collagenabscheidungen in der Haut; Verbesserung der Hauttextur und/oder Glätte und/oder Festigkeit, wobei das Verfahren das Auftragen einer lokal anzuwendenden Zusammensetzung, umfassend physikalisch behandeltes Wasser, welches ein Elektronenmangel aufweist, auf die Haut umfasst.

Besonders vorteilhaft ist es dabei, wenn das Aufbringen des physikalisch behandelten Wassers mittels eines Gels und/oder einer Creme und/oder einer Dispersion und/oder einer Suspension und/oder Lotion und/oder Schaum und/oder Mousse erfolgt.

Als weiterhin vorteilhaft hat sich ein Verfahren zur Übertragen eines Elektronenmangels auf die Haut zur physikalischen der Haut zum Erzielen eines Hautpflegevorteils, ausgewählt aus: Behandeln/Verhindern von faltiger, herunterhängender und/oder geltender Haut; Verstärken der Collagenabscheidungen in der Haut; Verbesserung der Hauttextur und/oder Glätte und/oder Festigkeit, wobei das Verfahren zur Auftragen einer lokal anzuwendenden Zusammensetzung, umfassend physikalisch behandeltes Wasser, welches ein Elektronenmangel aufweist, auf die Haut umfasst, erwiesen.

Es ist seit langem anerkannt, dass es ein normales transkutanes Potential gibt, das an der Säugerhaut anliegt (s. z.B. : Robert Edelberg, in : The Biophysical Properties ofthe Scin, Harry Elden (Hrsg., Kap. 15, Wiley Interscience, 1971). Dieses Potential wird zu einem großen Teil von der Anwesenheit von Schweißdrüsen und Haaren beeinflusst, und daher kann die Stärke des Potentials auf der Haut sowohl örtlich als auch zeitlich variieren. Jedoch gibt es auch in non-glandulären Regionen der Haut einen recht starken messbaren Strom, der über die Epidermis aufgebaut wird.

Es gibt Hinweise darauf, dass die Aufrechterhaltung eines elektrischen Stroms auf der Haut mit dem fortdauernden Wohlergehen unbeschädigter Haut in Verbindung steht, und dass das Anlegen eines Stroms an verletzter Haut sehr vorteilhaft für den Heilungsprozess beschädigter Haut sein kann. Auch erscheint eine potentiell präventive und/oder therapeutische Anwendung eines Stroms mit niedriger Intensität vorstellbar. Die Mittel zur Verabreichung eines heilenden bzw. pflegenden Stroms umfassen jedoch typischerweise die Verwendung von Maschinen, was jedoch teuer und kompliziert und darüber hinaus mitunter gefährlich ist, uns somit für die Pflege bzw. Behandlung von Hautirritationen bzw. leichten Störungen oder Erkrankungen nicht geeignet ist. Es ist daher wünschenswert, ein weniger intrusives, dafür aber kosteneffektives Verfahren zur Verfügung zu haben, um die Gesamtgesundheit der Haut aufrechtzuerhalten.

Die Matrix, in welcher die Komponenten aufgebracht bzw. angewendet werden, kann jeder kosmetisch oder pharmazeutisch annehmbare Trägerstoff sein. Der Begriff "pharmazeutisch oder kosmetisch annehmbare Trägerstoff' bezieht sich auf einen Trägerstoff, für entweder pharmazeutische oder kosmetische Verwendung, welcher die aktiven Komponenten am beabsichtigten Zielort bereitstellt und welcher Mensch oder anderen Empfängermechanismen keinen Schaden zufügt.

Die Zusammensetzungen können in jeder Form präpariert werden, die sich zum topischen Auftragen auf die Haut eignen. Solche Formen umfassen, ohne Einschränkung auf diese, Gels, Cremes, Dispersionen, (Wasser-in-Öl oder Öl-in-Wasser), Suspensionen, Lotionen, Schäume, Mousse und ähnliche. Zusätzlich kann das Verfahren bzw. die Zubereitung als Hilfsmittel für den Wundheilungsprozess verwendet werden. Wie oben gezeigt ist bekannt, dass heilende Haut mit einem messbar erhöhten Strom einhergeht. Die erfindungsgemäße Zusammensetzung bzw. das erfindungsgemäße Verfahren bzw. die erfindungsgemäße Verwendung können verwendet werden, um den natürlich auftretenden Prozess zu verbessern bzw. zu verstärken, entweder durch direkte Kombination mit Wundheilenden Wirkstoffen, oder allein in getrennten Anwendungen.

Das physikalisch behandelte Wasser wird wie folgt gewonnen:
Basierend auf der Erzeugung einer Influenz in einem elektrischen Feld (elektrostatische Induktion) werden im Wasser Ladungsträger separiert und negative Ladungsträger zu einem gewissen Teil abgeführt. Aufgefangen wird schließlich Fraktion mit einen positiven elektrostatischen Ladung. Auf diese Weise können die positiv geladenen Ladungsträger weitergegeben werden, so dass sie eine starke Elektronenakzeptanz der Flüssigkeit bewirkten.

Dem Wasser wird durch den unten beschriebenen Mechanismus Elektronen entzogen. Ein Elektronenmangel in den Clustern (unsauber als Proton bezeichnet) führt nicht zur Instabilität des Clusterverbandes, sondern wird über den Grothus Mechanismus (1820) durch sogenanntes Proton-Hopping ausgeglichen.

Das in dem erfindungsgemäßen Verfahren verwendete physikalisch behandelte Wasser wird durch elektrostatische Induktion bzw. Influenz gewonnen. Dabei führt eine Bewegung im elektrischen Feld zur Trennung von Ladung, der Influenz.

Clusterwasser, dessen Leitfähigkeit durch den Zusatz von Kochsalz auf einen Sollwert voreingestellt ist, fließt in einen elektrostatischen Feld (ruhendes elektrisches Feld), das durch eine Anode und eine Katode gebildet wird.

Im ersten Schritt kommt es zum Ausrichten der Ladungen und freien Elektronen im elektrischen Feld.

Durch die Bewegungen und daraus folgende Influenz werden in einem zweiten Schritt die Ladungen getrennt und abgeführt.

Die Fraktion mit einem Mangel an Elektronen wird als zunutzendes Konzentrat abgeführt und gesammelt, das Ergebnis ist eine de-elektronisierte (positiv geladene) Fraktion.

Man erhält ein elektrostatisch positiv geladenes Wasser. Es hat einen hohen Bedarf, die ungeladenen Positionen in die Cluster zu füllen. Im Kontakt mit elektronenreichen Oberflächen gibt es einen elektrischen Schlag, der zum Ladungsausgleich führt.

Mit den nun folgenden Beispielen wird die Wirksamkeit des Verfahrens zur Erzielung eines Hautpflegevorteils veranschaulicht. Die Beispiele dienen der Veranschaulichung der Erfindung und sind nicht in einschränkendem Sinne zu verstehen.

In einer weiteren Ausführungsform umfasst die Erfindung ein mittels Influenzverfahren behandeltes Wasser als Arzneimittel, die Verwendung eines mittels Influenzverfahren behandelten Wassers zur Herstellung eines Arzneimittels sowie die Verwendung eines mittels Influenzverfahren behandelten Wassers als Arzneimittel, sowie als weitere Ausführungsform die Verwendung als Arzneimittel zur Behandlung von Erkrankungen der Haut oder des Auge.

### Beispiel 1

### Verfahren des Tests zur Verbesserung von rauer Haut

Die Proben der rauen Hautfläche wurden durch Auflegen von Filmen, welche eine 10%-ige Lösung Natriumlaurylsulfat absorbiert hatten, auf 3 cm² Flächen menschlicher Unterarmenhaut, Entfernen der Filme 6 Stunden später und Waschen der Flächen mit Wasser hergestellt. Anschließend wurde die physikalisch behandelte Flüssigkeit durch dreimal tägliches 5 minütiges Baden des Unterarmes in physikalisch behandeltem Wasser mit einer Temperatur von 28 bis 32 C° in Kontakt mit der Haut gebracht. Nach 4 bzw. 8 Tagen wurden die rauen Hauttestflächen mit dem bloßen Auge untersucht, und anschließend wurden die Hornhautfeuchtigkeitsgehalte gemessen, um die Verbesserung mit einem Hautoberflächenhornhautfeuchtigkeitsmessgerät, SKICON-200 (IBS Corporation) zu überprüfen. Die Ergebnisse sind in den Tabellen 1 und 2 gezeigt.

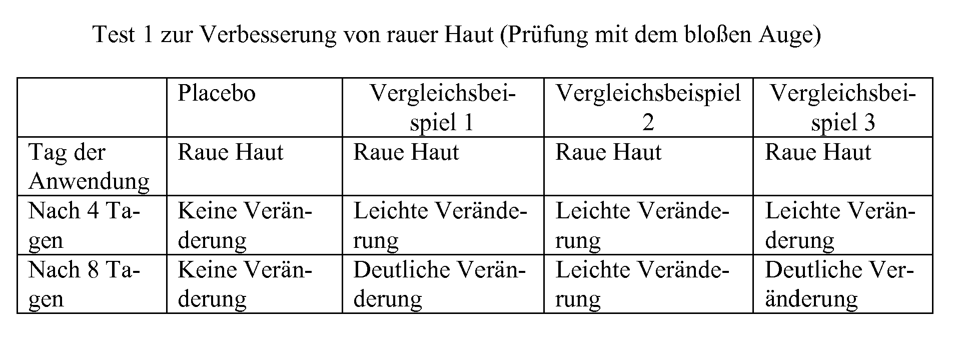

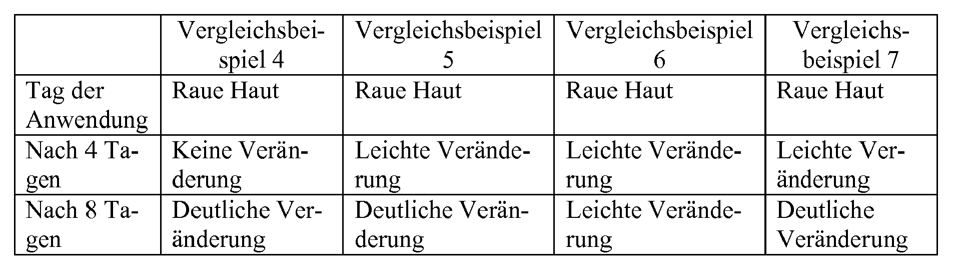

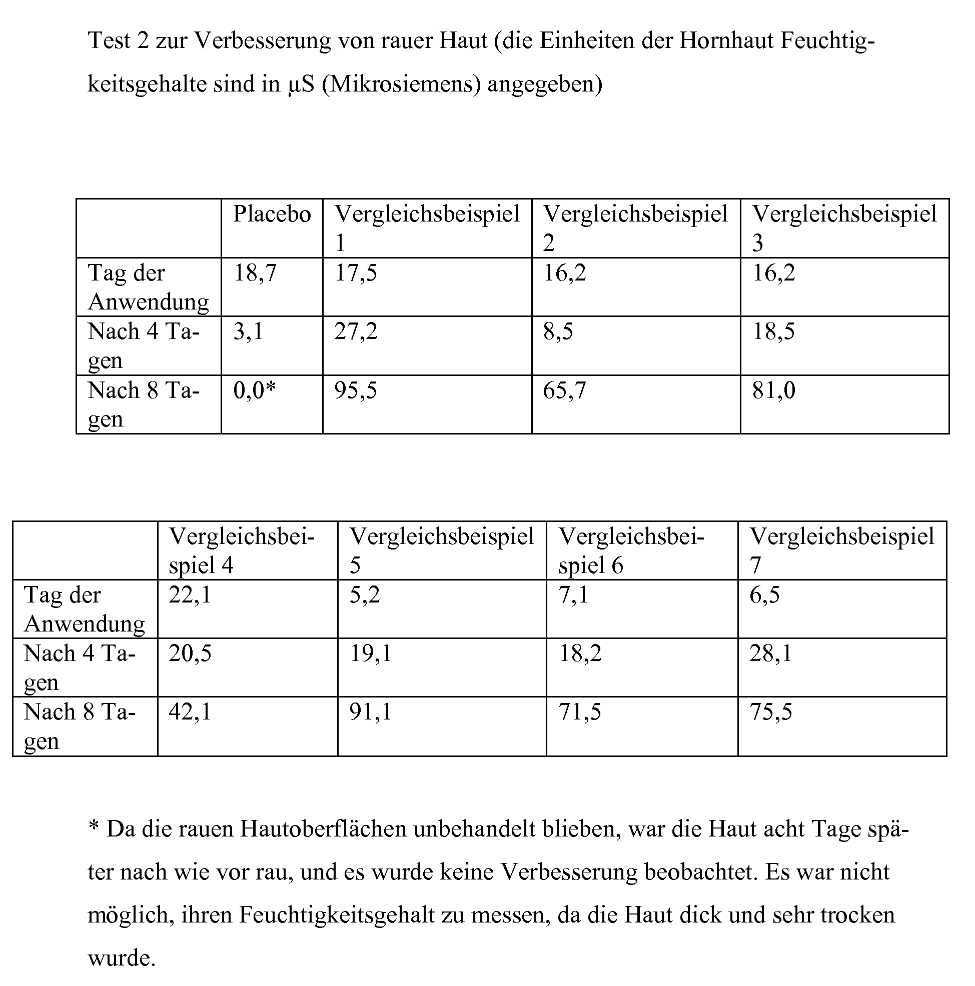

### Beipiel 2:

Eine pharmazeutisch annehmbare Zusammensetzung zur Verabreichung von physikalisch behandeltem Wasser weist folgende Zusammensetzung auf:

### Zusammensetzung der Salbengrundlage "Wasserhaltige hydrophile Salbe":

### Bestandteile

| | |
|---|---|
| Emulgierender Cetylstearylalkohol, Typ A | 9,0 g |
| Dickflüssiges Paraffin | 10,5 g |
| Weißes Vaselin | 10,5 g |
| Physikalisch behandeltes Wasser | 50 g |
| Gereinigtes Wasser | ad 100,0 g |

### Zusammensetzung der Zubereitung zur kosmetischen Anwendung:

| | |
|---|---|
| Harnstoff | 7,5 T |
| Milchsäure (90% m/m) | 1,0 T |
| Natriumlactat-Lösung (50% m/m) | 4,0 T |
| Wasserhaltige Hydrophile Salbe (s. o.) | ad 100,0 T |

Von dieser Rezeptur sind 30g herzustellen und entsprechend zu verabreichen.

Nach Bedarf und Beschaffenheit der Haut kann auch die Salbengrundlage "wasserhaltige hydrophile Salbe" alleine aufgetragen werden.

Die Erfindung bietet eine erhebliche Verbesserung bei der Hautpflege und Unterstützung der natürlichen Hautfunktionen.

## Patentansprüche

1. Kosmetisches Verfahren zur Erzielung eines Hautpflegevorteils, ausgewählt aus: Behandeln/Verhindern von faltiger, herunterhängender und/oder gealterter Haut; Verstärken der Collagenabscheidungen in der Haut; Verbesserung der Hauttextur und/oder Glätte und/oder Festigkeit; Reinigung der Haut, wobei das Verfahren das Auftragen einer lokal anzuwendenden Zusammensetzung, umfassend physikalisch behandeltes Wasser, auf die Haut umfasst, wobei das physikalisch behandelte Wasser mittels eines Influenzverfahrens gewonnen wird.

2. Kosmetisches Verfahren nach Anspruch 1, **dadurch gekennzeichnet, dass** das physikalisch behandelte Wasser einen Elektronenmangel aufweist.

3. Kosmetisches Verfahren nach Anspruch 1 oder 2, **dadurch gekennzeichnet, dass** das physikalisch behandelte Wasser auf die Haut aufgetragen und/oder eingerieben und/oder aufgesprüht und/oder mittels Eintauchen des Körpers bzw. Körperteils in das physikalisch behandelte Wasser erfolgt.

4. Kosmetisches Verfahren nach einem der Ansprüche 1 bis 3, **dadurch gekennzeichnet, dass** das Influenzverfahren bei einer Stromdichte von 0,5 bis 10 W pro cm2 durchführbar ist.

5. Kosmetisches Verfahren nach einem der vorhergehenden Ansprüche, **dadurch gekennzeichnet, dass** das Influenzverfahren folgende Schritte umfasst: Einbringen des zu behandelnden Wassers in ein elektrisches Feld, Ausrichtung der Ladungen und freien Elektronen im elektrischen Feld, Trennung der Ladungen durch Bewegung und der daraus resultierenden Influenz und Sammlung und Abführung der de-elektronisierten, positiv geladenen Fraktion.

6. Kosmetisches Verfahren nach einem der vorhergehenden Ansprüche, **dadurch gekennzeichnet, dass** das Aufbringen des physikalisch behandelten Wassers mittels eines Gel und/oder Creme und/oder Dispersion und/oder Suspension und/oder Lotion und/oder Schaum und/oder Mousse erfolgt.

7. kosmetisches Verfahren nach einem der vorhergehenden Ansprüche, **dadurch gekennzeichnet, dass** das Verfahren zur Pflege von Schleimhäuten eingesetzt wird, insbesondre der Mundhöhle und des Auges.

8. Verwendung von physikalisch behandeltem Wasser zur Herstellung einer lokal anzuwendenden Zusammensetzung zur Verwendung in einem Verfahren nach einem der vorhergehenden Ansprüche.

9. Verwendung nach Anspruch 8, **dadurch gekennzeichnet, dass** die lokal anzuwendende Zusammensetzung ein Gel und/oder Creme und/oder Dispersion und/oder Suspension und/oder Lotion und/oder Schaum und/oder Mousse umfasst.

10. Verfahren zur Übertragung eines Elektronenmangels auf die Haut zur physikalischen Behandlung der Haut zur Erzielung eines Hautpflegevorteils, ausgewählt aus: Behandeln/Verhindern von faltiger, herunterhängender und/oder gealterter Haut; Verstärken der Collagenabscheidungen in der Haut; Verbesserung der Hauttextur und/oder Glätte und/oder Festigkeit, wobei das Verfahren das Auftragen einer lokal anzuwendenden Zusammensetzung, umfassend physikalisch behandeltes Wasser, welches einen Elektronenmangel aufweist, auf die Haut umfasst.

11. Verfahren nach Anspruch 10, **dadurch gekennzeichnet dass** das physikalisch behandelte Wasser in ein Gel und/oder Creme und/oder Dispersion und/oder Suspension und/oder Lotion und/oder Schaum und/oder Mousse gemischt wird.
